# EUROPEAN PATENT APPLICATION

(11) **EP 2 985 605 A1**
(43) Date of publication of application: **17.02.2016**
(21) Application number: 14782540.0
(22) Date of filing: 19.03.2014
(51) Int. Cl.: G01N 33/574, G01N 33/53, C07K 16/28, C12P 21/08

(54) **METHOD FOR DETECTING COLON CANCER**

(30) Priority: 08.04.2013 JP 2013080303
(71) Applicant: Shionogi & Co., Ltd., Osaka-shi, Osaka 541-0045 (JP); Theoria Science Inc., Tokyo 101-0032 (JP)
(72) Inventor: OHTA, Hideki, Osaka 561-0825 (JP); OKAMOTO, Hiroyuki, Osaka 561-0825 (JP); SONODA, Hikaru, Osaka 566-0022 (JP); OCHIYA, Takahiro, Tokyo 104-0045 (JP)
(74) Representative: Denison, Christopher Marcus
(86) International application number: PCT/JP2014/057588
(87) International publication number: WO 2014/167969

(57) **Abstract**

A method for detecting a colorectal cancer, including the steps of measuring an amount of exosomes expressing CD 147 in a body fluid sample derived from a test individual with an anti-CD 147 monoclonal antibody or a fragment thereof; and comparing a signal intensity of exosomes in the step with a signal intensity in a control individual, wherein a case where the signal intensity in the test individual is found to be stronger than the signal intensity in the control individual serves as an index of the presence of the colorectal cancer. According to the method of the present invention, whether or not a sample provider has a high possibility of developing a colorectal cancer can be judged. Therefore, the method is useful because the sample provider can take a means of inhibiting the progression of cancer.

## Description

### TECHNICAL FIELD

The present invention relates to a method for detecting a colorectal cancer. More particularly, the present invention relates to a method for detecting the presence of a colorectal cancer with a monoclonal antibody or an antibody fragment thereof against a particular antigen (CD9, CD63, or CD 147) on an exosomal surface in samples, a method for evaluating a therapeutic effect to a colorectal cancer, and a kit used in these methods.

### BACKGROUND ART

An exosome is a granular vesicle existing in a body fluid in a living body. It has been known that a wide variety of membrane proteins exist on an exosomal surface, as in the case with a general cell surface. In addition, it has been reported that exosomes are secreted from various kinds of cells, for example, cells of the immune system and various cancer cells, and the function as an intermediary in intercellular communication in a living body to be associated with physiological phenomena and association with a disease such as cancer have been remarked.

For example, it has been reported in Non-Patent Publication 1 that exosomes are isolated from ascites or blood of ovarian carcinoma patients, and exosomal uptake by immune cells results in suppression of immunization system, leading to augmented tumor growth. In the detection of exosomes, antibodies against CD24, ADAM10, and CD9 have been used.

In addition, Patent Publication 1 discloses that various cancers are diagnosed with CD9, CD31, CD63, CD81, CD82, CD37, CD53 or the like as a surface marker for vesicles existing in live bodies. Non-Patent Publication 2 discloses that HAb18G/CD147 is expressed in a higher level in 28 kinds of carcinoma cells than normal cells, so that a monoclonal antibody against the protein can be used as a tumor biomarker. Non-Patent Publication 3 has reported that expression of MAGE-1 and HER-2/neu is increased in microvesicles existing in plasma of patients with gastric cancer. Non-Patent Publication 4 discloses a kit for diagnosing cancer antigen by measuring CA19-9 in sera.

### PRIOR ART REFERENCES

### PATENT PUBLICATIONS

Patent Publication 1: WO 2012/115885

### NON-PATENT PUBLICATIONS

Non-Patent Publication 1: Sascha Keller, et al., Cancer Letters, 2009, 278, 73-81
Non-Patent Publication 2: Yu Li, et al., Histopathology, 2009, 54, 677-687
Non-Patent Publication 3: Jaroslaw Baran, et al., Cancer Immunol Immunother, 2010, 59, 841-850
Non-Patent Publication 4: Package Insert of AxSYM (registered trademark) CA 19-9 Dynapack (registered trademark), ABBOTT JAPAN CO., LTD.

### SUMMARY OF THE INVENTION

### PROBLEMS TO BE SOLVED BY THE INVENTION

It is possible to detect cancer using a membrane protein existing in a granular vesicle such as exosomes as a biomarker. However, it is difficult to obtain satisfactory results for the reasons that the fluctuations of sensitivities and specificities are large depending upon the kinds of samples and antibodies used, so that some cases are judged to be pseudo-positive in the conventional cancer diagnosis, and have some disadvantages in diagnostic accuracy. Accordingly, the development of further techniques is needed in order to detect cancer accurately by detecting exosomes.

An object of the present invention is to provide a method for detecting a colorectal cancer including measuring exosomes, and a kit used in the method.

### MEANS TO SOLVE THE PROBLEMS

The present invention relates to the following [1] to [7]:
[1] A method for detecting a colorectal cancer, including the steps of:
   measuring an amount of exosomes expressing CD 147 in a body fluid sample derived from a test individual with an anti-CD 147 monoclonal antibody or a fragment thereof; and
   comparing a signal intensity of exosomes in the above step with a signal intensity in a control individual,
   wherein a case where the above signal intensity in the test individual is found to be stronger than the signal intensity in the control individual serves as an index of the presence of the colorectal cancer.
[2] A method for detecting a colorectal cancer, including the steps of: measuring an amount of exosomes expressing CD9 and/or CD63, and CD 147 in a body fluid sample derived from a test individual with
   at least one member selected from the group consisting of an anti-CD9 monoclonal antibody and a fragment thereof and an anti-CD63 monoclonal antibody and a fragment thereof, and
   an anti-CD 147 monoclonal antibody or a fragment thereof; and comparing a signal intensity of exosomes in the above step with a signal intensity in a control individual,
   wherein a case where the above signal intensity in the test individual is found to be stronger than the signal intensity in the control individual serves as an index of the presence of the colorectal cancer.
[3] A method for evaluating treatment of a colorectal cancer, including the steps of:
   measuring an amount of exosomes expressing CD9 and/or CD63, and CD 147 in a body fluid sample derived from a test individual with at least one member selected from the group consisting of an anti-CD9 monoclonal antibody and a fragment thereof and an anti-CD63 monoclonal antibody and a fragment thereof, and
   an anti-CD 147 monoclonal antibody or a fragment thereof; and
   comparing a signal intensity of exosomes in the above step with a signal intensity in the test individual before the treatment,
   wherein the method includes the step of evaluating the treatment to have therapeutic effects to a colorectal cancer, in a case where the signal intensity after the treatment is found to be weaker than the signal intensity before the treatment.
[4] A kit for use in a method as defined in any one of the above [1] to [3], wherein the kit contains an anti-CD147 monoclonal antibody or a fragment thereof.
[5] A kit for use in the method as defined in the above [2] or [3], wherein the kit contains an anti-CD9 monoclonal antibody and/or an anti-CD63 monoclonal antibody or fragments thereof, and an anti-CD 147 monoclonal antibody or a fragment thereof.
[6] Use of exosomes as a marker of a colorectal cancer, recognized by
   at least one member selected from the group consisting of an anti-CD9 monoclonal antibody and a fragment thereof and an anti-CD63 monoclonal antibody and a fragment thereof, and
   an anti-CD 147 monoclonal antibody or a fragment thereof.
[7] A method for providing information for a colorectal cancer or a suspect of a colorectal cancer, characterized in that the method includes detecting exosomes recognized by
   at least one member selected from the group consisting of an anti-CD9 monoclonal antibody and a fragment thereof and an anti-CD63 monoclonal antibody and a fragment thereof, and
   an anti-CD 147 monoclonal antibody or a fragment thereof from a body fluid sample derived from a test individual.

### EFFECTS OF THE INVENTION

According to the method of the present invention, a colorectal cancer can be detected by measuring exosomes without the concerns of being pseudo-positive, whereby the presence or absence of the onset of the colorectal cancer or the progressive degree can be judged.

### BRIEF DESCRIPTION OF THE DRAWINGS

[FIG. 1] FIG. 1 is a view showing the results of proteomic analysis of exosomes derived from a human colorectal cancer cell line HCT116.
[FIG. 2] FIG. 2 is a view showing the results of detection of CD 147 using a Western blot method, in which 500 ng per lane of an exosomal extract is electrophoresed, and detection is carried out using an anti-CD 147 mouse monoclonal antibody.
[FIG. 3] FIG. 3 is a graph showing the results of detection of CD 147 in exosomes derived from a colorectal cancer cell line using an ExoScreen method. Biotinylated antibody: CD 147, acceptor beads conjugated antibody: CD9.
[FIG. 4] FIG. 4 is a graph showing the results of detection of CD 147 in exosomes derived from sera of patients with a colorectal cancer using an ExoScreen method. Biotinylated antibody: CD 147, acceptor beads conjugated antibodies: CD63 (left panel), and CD9 (right panel).
[FIG. 5] FIG. 5 is a graph showing the results of detection of CD 147 in exosomes derived from sera of patients with a colorectal cancer using an ExoScreen method. Biotinylated antibody: CD 147, acceptor beads conjugated antibody: CD9.
[FIG. 6] FIG. 6 is a view showing comparisons of the results of detection of each of CD 147, CEA, and CA19-9 derived from exosomes in sera of patients with a colorectal cancer.
[FIG. 7A] FIG. 7A is a graph showing the results of ROC analysis in a case of diagnosing a colorectal cancer using CD 147 derived from exosomes as an index (FIG. 5).
[FIG. 7B] FIG. 7B is a graph showing the results of ROC analysis in a case of diagnosing a colorectal cancer using CEA as an index.
[FIG. 7C] FIG. 7C is a graph showing the results of ROC analysis in a case of diagnosing a colorectal cancer using CA19-9 as an index.
[FIG. 8] FIG. 8 is a graph showing the results of detection of CD 147 in exosomes derived from sera of patients with a colorectal cancer before and after the operation using an ExoScreen method. The central line of each of the data boxes shows the detection results of a median value. Biotinylated antibody: CD 147, acceptor beads conjugated antibody: CD9.

### MODES FOR CARRYING OUT THE INVENTION

The present invention is a method for detecting a colorectal cancer in a test individual, and the invention has a great feature in that the method includes measuring a signal derived from exosomes in a body fluid sample with a specific monoclonal antibody, and judging that it is possible that an individual is suffering from a colorectal cancer in a case where the value is greater than that of a normal individual. Concretely, the method includes measuring an amount of exosomes expressing a specific antigen in a body fluid sample derived from a test individual with a monoclonal antibody or a fragment thereof against the antigen (hereinafter also referred to as "step A"); and comparing a signal intensity of exosomes in the above step with a signal intensity in a control individual (hereinafter also referred to as "step B"), wherein a case where the above signal intensity in the test individual is found to be stronger than the signal intensity in the control individual serves as an index of the presence of the colorectal cancer. Here, the detection of a colorectal cancer as used herein embraces the detection of the presence or absence of onset of a colorectal cancer, or a progressive degree of pathology.

The present inventors have previously found that according to a measurement system composed of a combination of an anti-CD9 antibody and an anti-CD63 antibody, a stronger signal intensity is obtained in the blood of patients with a colorectal cancer than the blood of normal individuals. However, there are some disadvantages that strong signals are detected to some extent even in normal individuals, so that it is disadvantageous as a diagnostic method of a colorectal cancer. However, in the present invention, identification of CD 147 as an antigen specific to exosomes secreted from colorectal cancer cells having high malignancy was succeeded. Further, as a result of intensive studies, the present inventors have found that the fluctuations of the measurement values of the normal individuals are small without detecting false positive values by combining an antibody against this CD 147 antigen with an anti-CD9 antibody or an anti-CD63 antibody, whereby obtaining excellent results that the signals are detected only from the patients with a colorectal cancer. The present invention is perfected thereby.

Each of the steps in the present invention will be explained hereinbelow.

The step A is a step of measuring an amount of exosomes expressing a specific antigen in a body fluid sample derived from a test individual with a monoclonal antibody or a fragment thereof against the antigen.

The monoclonal antibody or a fragment thereof used in the step A include 3 kinds of monoclonal antibodies or fragments thereof. Concretely, an anti-CD 147 monoclonal antibody or a fragment thereof, an anti-CD9 monoclonal antibody or a fragment thereof, and an anti-CD63 monoclonal antibody or a fragment thereof are used, and at least an anti-CD 147 monoclonal antibody or a fragment thereof is used.

Each of the monoclonal antibody or a fragment thereof used in the present invention may be one recognizing a specific antigen, and can be prepared in accordance with a known method. In other words, an anti-CD 147 monoclonal antibody or a fragment thereof recognizes CD 147, an anti-CD9 monoclonal antibody or a fragment thereof recognizes CD9, and an anti-CD63 monoclonal antibody or a fragment thereof recognizes CD63, which may be prepared in accordance with immune responses of mammals, or may be prepared on the basis of the sequence information of each antigen.

Also, in the present invention, as the anti-CD9 monoclonal antibody and the anti-CD63 monoclonal antibody, ones obtained from cells deposited at International Patent Organism Depositary, National Institute of Technology and Evaluation, Incorporated Administrative Agency (Tsukuba Central 6, 1-1-1 Higashi, Tsukuba, Ibaraki-ken, Japan) under the accession numbers given hereinbelow can also be used as a hybridoma producing the above monoclonal antibodies:
FERM BP-11519 (the monoclonal antibody produced is a CD9-12A12 antibody, identification: CD9:12A12, date of receipt: November 8, 2011) FERM BP-11520 (the monoclonal antibody produced is a CD63-8A12 antibody, identification: CD63:8A12, date of receipt: November 8, 2011) FERM BP-11521 (the monoclonal antibody produced is a CD63-13C8 antibody, identification: CD63:13C8, date of receipt: November 8, 2011)

In the present invention, a "monoclonal antibody fragment" means a part of a monoclonal antibody mentioned above, the fragment having a specific binding property to CD9, CD63 or CD 147 in the same manner as in the monoclonal antibody. The fragment having a specific binding property to CD9, CD63 or CD147 concretely includes Fab, F(ab')₂, Fab', a single-chain antibody (scFv), a disulfide-stabilized antibody (dsFv), a dimerized V region fragment (Diabody), peptides including CDR, and the like *(*Expert Opinion on Therapeutic Patents, 6(5), 441-456, 1996).

These monoclonal antibodies or fragments thereof are used, for example in the following two embodiments.
Embodiment 1: an embodiment using an anti-CD 147 monoclonal antibody or a fragment thereof
Embodiment 2: an embodiment using in combination
at least one member selected from the group consisting of an anti-CD9 monoclonal antibody and a fragment thereof and an anti-CD63 monoclonal antibody or a fragment thereof, and
an anti-CD 147 monoclonal antibody or a fragment thereof

In Embodiment 1, CD 147 on exosomes can be recognized with a labeled anti-CD 147 monoclonal antibody or a fragment thereof to quantify exosomes expressing CD147. The labeling of the anti-CD 147 monoclonal antibody or a fragment thereof is not particularly limited, and the labeling can be carried out in accordance with a known method. The monoclonal antibody or a fragment thereof of Embodiment 1 is suitably used in a Western blot method described later.

In Embodiment 2, CD9 and/or CD63 and CD 147 on exosomes can be recognized with monoclonal antibodies or fragments against these antigens to quantify exosomes expressing CD9 and/or CD63 and CD147. In Embodiment 2, the anti-CD 147 monoclonal antibody or a fragment thereof may be used as an immobilized antibody, and the anti-CD9 monoclonal antibody or a fragment thereof may be used as a labeled antibody. Alternatively, the anti-CD9 monoclonal antibody or a fragment thereof may be used as an immobilized antibody, and the anti-CD 147 monoclonal antibody or a fragment thereof may be used as a labeled antibody. In addition, the anti-CD63 monoclonal antibody or a fragment thereof may be used as an immobilized antibody, and the anti-CD 147 monoclonal antibody or a fragment thereof may be used as a labeled antibody. Alternatively, the anti-CD63 monoclonal antibody or a fragment thereof may be used as a labeled antibody, and the anti-CD 147 monoclonal antibody or a fragment thereof may be used as an immobilized antibody. Also, the anti-CD9 monoclonal antibody or a fragment thereof and the anti-CD63 monoclonal antibody or a fragment thereof may be used as an immobilized antibodies, and the anti-CD 147 monoclonal antibody or a fragment thereof may be used as a labeled antibody. Alternatively, the anti-CD9 monoclonal antibody or a fragment thereof and the anti-CD63 monoclonal antibody or a fragment thereof may be used as labeled antibodies, and the anti-CD 147 monoclonal antibody or a fragment thereof may be used as an immobilized antibody. The preparation of the an immobilized antibodies and the labeled antibodies are not particularly limited, and the antibodies can be prepared in accordance with known methods. The monoclonal antibody or fragment thereof of Embodiment 2 is suitably used in a sandwich ELISA method, or an ExoScreen method described later.

Samples to be used for measuring the amount of exosomes in the present invention are not particularly limited so long as the samples are body fluid samples, and, for example, exemplified by ones selected from the group consisting of blood, sera, plasma, urine, saliva, milk, nasal discharge, and cerebrospinal fluid.

The measurement of the amount of exosomes may be any methods so long as the methods use the monoclonal antibody or a fragment thereof mentioned above, and the measurement can be carried out, for example, in accordance with a Western blot method, a sandwich ELISA method, and an ExoScreen method.

In the Western blot method, for example, the monoclonal antibody or a fragment thereof of Embodiment 1 can be used. Concretely, CD 147 existing on exosomes derived from a colorectal cancer cell can be detected by analyzing blood of patients with a colorectal cancer in accordance with a Western blot method with an anti-CD 147 monoclonal antibody or a fragment thereof.

In the sandwich ELISA method, for example, the monoclonal antibody or a fragment thereof of Embodiment 2 can be used. Concretely, first, one kind of a monoclonal antibody or a fragment thereof is used as a solid phase antibody, and the solid phase antibody is contacted with a sample containing exosomes to form a complex. Thereafter, another monoclonal antibody or a fragment thereof previously labeled is added thereto, to form a further complex, whereby an amount of exosome expressing antigens recognized by both the antibodies can be measured by detecting a label.

The ExoScreen method is a method to which AlphaLISA developed by PerkinElmer is applied. The present method uses two kinds of antibodies having different epitopes, wherein one antibody is biotinylated, and the other is conjugated to AlphaLISA acceptor beads to react with an analyte sample. Thereafter, streptavidin-conjugated donor beads are added thereto to conjugate the biotinylated antibody with the donor beads via streptavidin, so that acceptor beads are adjoining to donor beads. In an adjoining state (within 200 nm), the donor beads are excited at 680 nm, resulting in the release of singlet oxygen from the donor beads, and light at 615 nm is emitted when the singlet oxygen reaches the acceptor beads, which can be detected as a signal. By applying this method to the monoclonal antibody or a fragment thereof of Embodiment 2, exosomes having sizes about 100 nm can be measured as analyte samples.

Thus, the amount of exosomes containing a target protein in a body fluid sample can be measured. The subsequent step B is carried out using the amount of exosomes obtained.

The step B is a step of comparing a signal intensity of the exosomes obtained in the step A with a signal intensity in a control individual, wherein a case where the above signal intensity in the test individual is found to be stronger than the signal intensity in the control individual serves as an index of the presence of the colorectal cancer. The control individual as used herein may be any individuals not developing a colorectal cancer, and include normal individuals.

The signal intensity in the control individual is an amount of exosomes in a body fluid sample derived from the control individual, and the signal intensity may be measured together when the amount of exosomes of the test individuals is measured in the step A, or measured separately. In addition, the amounts of exosomes of a plurality of control individuals are measured, and the amounts of exosomes of the control individual may be set from the statistics thereof.

The body fluid sample derived from the control individuals is preferably the same kinds of samples as the body fluid sample derived from test individuals. For example, in a case where a body fluid sample derived from a test individual is blood, a body fluid sample derived from a control individual is also blood.

In order to compare the signal intensity in the test individual with the signal intensity of the control individual, the amount of exosomes of the test individual and the amount of exosomes of the control individual are compared and analyzed. The method for comparison is not particularly limited, and a known method (a Steel method, a t-test, a Wilcoxon test or the like) can be used. When it is shown that the amount of exosomes of the test individual is significantly increased as compared to the amount of exosomes of the control individual according to the above analysis, it is judged that it is highly possible that a colorectal cancer is present in the test individual.

Here, since the presence or absence of potential existence of a colorectal cancer can be judged by detecting exosomes existing in a body fluid sample using the above antibodies, one embodiment of the present invention includes a method for providing information of a colorectal cancer or suspect of a colorectal cancer, characterized in that the method includes detecting exosomes recognized by
at least one member selected from the group consisting of an anti-CD9 monoclonal antibody and a fragment thereof and an anti-CD63 monoclonal antibody and a fragment thereof, and
an anti-CD 147 monoclonal antibody or a fragment thereof from a body fluid sample derived from a test individual.

In addition, in the above analysis, when it is shown that the amount of exosomes after the operation is reduced by setting the amount of exosomes of a control individual as the amount of exosomes before the operation of a test individual, and comparing the amount of exosomes after the operation as the amount of exosomes of a test individual, it can be judged to be highly possible that the colorectal cancer is diminished or abated.

Furthermore, in the above analysis, in a case where a test individual is diagnosed to have a colorectal cancer, when it is shown that amount of exosome after the treatment is reduced by setting the amount of exosomes of the control individual as the amount of exosomes before the treatment of a test individual, and comparing the amount of exosomes after the treatment as the amount of exosomes of a test individual, it can be judged to be highly possible that the treatment is effective in the treatment of the colorectal cancer. Therefore, the present invention also can provide a method for evaluation characterized in that the method includes measuring signals derived from exosomes with the above monoclonal antibody or a fragment thereof before and after receiving the treatment of a colorectal cancer, and judging the treatment to have effects in a case where the values after the treatment are smaller than those before the treatment.

In addition, another embodiment of the present invention provides a kit for detecting a colorectal cancer.

The kit of the present invention includes all sorts, so long as exosomes in a body fluid sample can be detected. Concretely, the kit includes a kit containing an antibody that can recognize an antigen existing on the above exosomal surface, in other words, an anti-CD 147 monoclonal antibody or a fragment thereof, an anti-CD9 monoclonal antibody or a fragment thereof, and an anti-CD63 monoclonal antibody or a fragment thereof. Among them, the embodiments using these antibody or fragments thereof include:
Embodiment 1: an embodiment containing an anti-CD 147 monoclonal antibody or a fragment thereof; and
Embodiment 2: an embodiment containing in combination of
   at least one member selected from the group consisting of an anti-CD9 monoclonal antibody and a fragment thereof and an anti-CD63 monoclonal antibody or a fragment thereof, and
   an anti-CD 147 monoclonal antibody or a fragment thereof in combination.

These kits can be used in any detection methods so long as the detection methods use an antibody when detecting exosomes in a body fluid sample (for example, a Western blot method, an ELISA method, an ExoScreen method, or the like). Here, so long as the exosomes are detected, a protein other than exosomes may be simultaneously detected with the above antibody.

By using the kit of the present invention, in a case where for example, amounts of exosomes existing in blood samples of normal individuals and test individuals are measured and caused to have a significant difference in the expression levels in both, the decision and/or diagnosis of onset of the colorectal cancer in the test individuals can be performed.

### EXAMPLES

The present invention will be explained on the basis of Examples hereinbelow, which are illustrated to more fully understand the present invention, without intending to limit the scope of the present invention to these Examples and the like.

### Test Example 1 (Detection of Exosomes Derived from Colorectal Cancer Cell Line with CD147 Antibody)

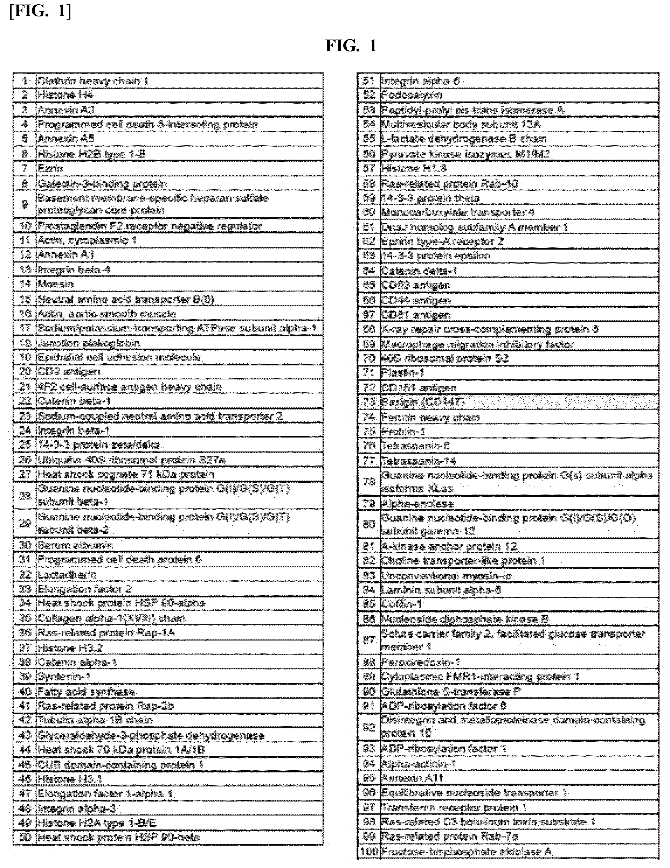

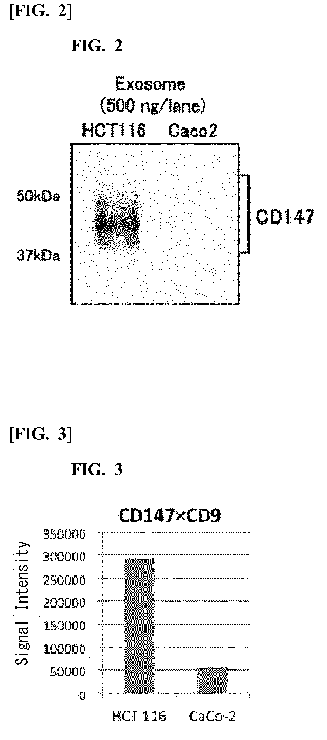

The results of proteomic analysis of exosomes derived from human colorectal cancer cell line HCT116 (American Type Culture Collection) are shown in FIG. 1. As to an antigen CD 147 extracted from the above results, expression of CD 147 in the exosomes derived from the colorectal cancer cell line was evaluated by Western blot method using a mouse antihuman CD 147 monoclonal antibody (manufactured by Novus Biologicals, Clone MEM-M 6/1). A comparison was made using HCT116 cells having very high malignancy and Caco2 cells (American Type Culture Collection) having low malignancy as the human colorectal cancer cell line. As a result, the presence of CD 147 could be confirmed with exosomes derived from HCT116 cell line having very high malignancy, but could not be detected with the exosomes derived from the Caco2 cells (FIG. 2).

Next, signals derived from exosomes expressing CD9 and CD 147 derived from each of colorectal cancer cell lines were measured by an ExoScreen method with the biotinylated anti-CD 147 monoclonal antibody (manufactured by Novus Biologicals, Clone MEM-M6/1) and acceptor beads conjugated anti-CD9 monoclonal antibody (monoclonal antibodies produced by hybridoma deposited under the accession number FERM BP-11519). As a result, expression of CD 147 in the exosomes derived from HCT116 cell line having high malignancy was confirmed (FIG. 3), in the same manner as the results according to the Western blot method. It is deduced from these results that exosomes expressing CD 147 are secreted in a part of the colorectal cancer cells in large amounts.

### Test Example 2 (Detection of Exosomes Derived from Sera of Colorectal Cancer Patients with CD147 Antibody-1)

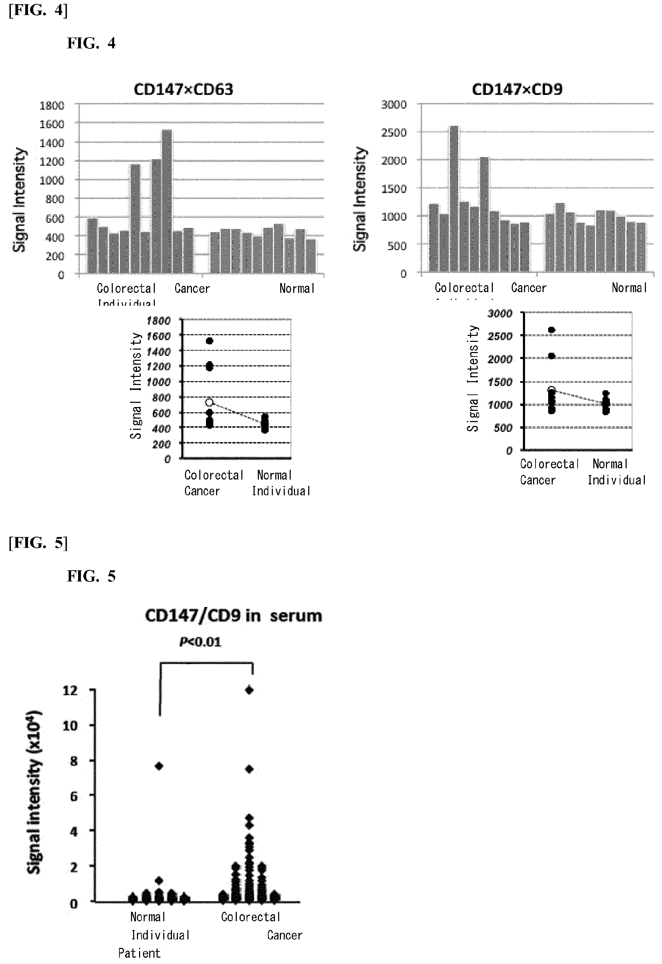

Signal intensity derived from exosomes expressing CD9 or CD63, and CD 147 in 5 µL of sera of colorectal cancer patients was detected by an ExoScreen method. In the ExoScreen method, a biotinylated anti-CD 147 monoclonal antibody used in Test Example 1 and acceptor beads conjugated anti-CD63 monoclonal antibody (monoclonal antibody produced by hybridoma deposited under the accession number FERM BP-11520) or the above acceptor beads conjugated anti-CD9 monoclonal antibody were used. Ten cases of sera derived from colorectal cancer patients and 10 cases of sera derived from normal individuals were analyzed. As a result, high signals were detected from the sera derived from 5 cases out of 10 cases of the patients. In these analyses, the rate of false-negative values was 50%, with small fluctuations of found values of normal individuals with completely no false-negative values, so that it was found to be excellent as the examination for colorectal cancer (FIG. 4).

### Test Example 3 (Detection of Exosomes Derived from Sera of Colorectal Cancer Patients with CD147 Antibody-2)

Signals derived from exosomes expressing CD9 and CD 147 were measured in the same manner as in Test Example 2, using sera derived from even more colorectal cancer patients and sera derived from normal individuals (FIG. 5). Concretely, 194 cases of sera derived from colorectal cancer patients and 191 cases of sera derived from normal individuals were used.

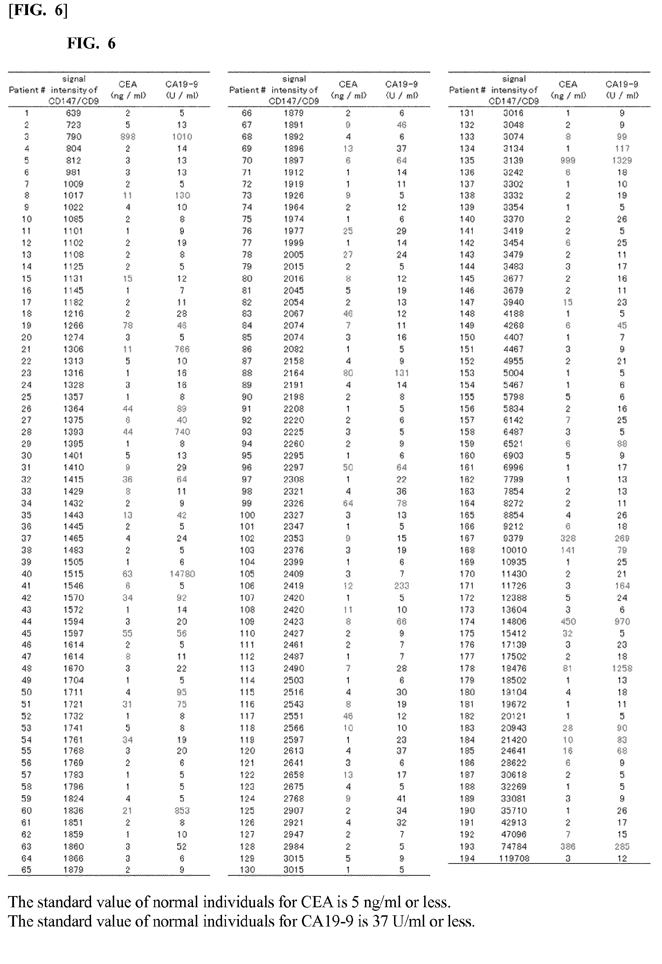

Next, the 194 cases of the same sera as the sera derived from colorectal cancer patients for which signals derived from exosomes were measured as mentioned above were subjected to measurements of CEA and CA19-9. The measurement results for CEA and CA19-9 and the results of measuring the signals derived from exosomes expressing CD9 and CD 147 in accordance with the ExoScreen method are collectively shown in FIG. 6.

It can be seen from FIG. 6 that when the measurement results of CEA and CA19-9 were compared, 33 cases were in common among 62 cases of CEA-positive cases and 36 cases of CA19-9-positive cases, and in these two methods, the same colorectal cancer patients can be detected with high correlations. On the other hand, no correlations were found between the results of measuring the signals derived from exosomes expressing CD9 and CD 147 in accordance with the ExoScreen method and the measurement results of CEA and CA19-9. In other words, it is suggested that different positive cases can be found by the combination of the examination with signals derived from exosomes expressing CD9 and CD147, with CEA or CA19-9, which is well-known in the colorectal cancer examination factor, so that even more colorectal cancer patients can be diagnosed.

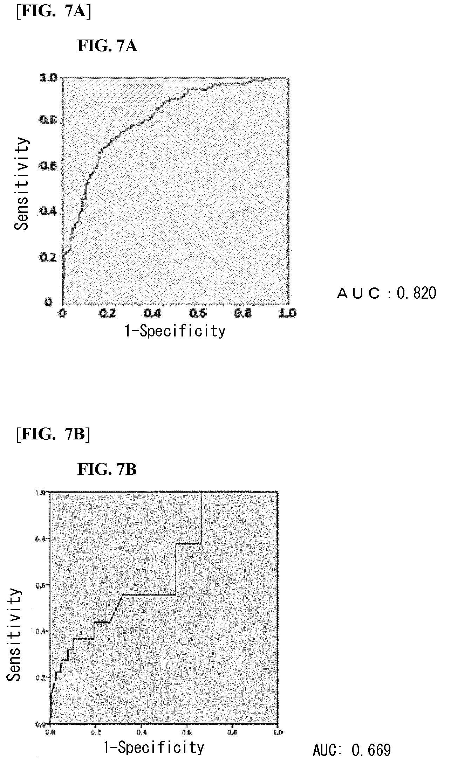

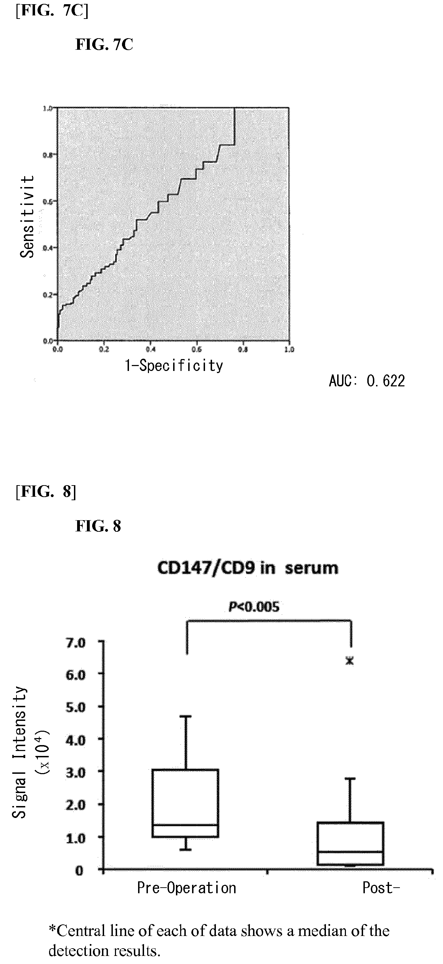

In addition, FIGs. 7A, 7B, and 7C show that ROC analysis was carried out to compare the detection abilities of the colorectal cancer in each of the measurement methods, where high detection ability is exhibited as the AUC approximates 1. In a case where the signals derived from exosomes expressing CD9 and CD 147 were used, the AUC was 0.820, which was found to be far larger than those of CEA or CA19-9 (0.669 and 0.622, respectively). It was shown that the examination of colorectal cancer using the signals derived from exosomes expressing CD9 and CD147 was more excellent than that of CEA or CA19-9.

### Test Example 4 (Analysis of Changes of Signal Intensities Before and After Operation)

The changes in the signals derived from the exosomes expressing CD9 and CD 147 before and after the operation were examined using sera derived from 15 patients subjected to excision operations of Stages I and II colorectal cancer (FIG. 8). It was found that the examination using signals derived from exosomes expressing CD9 and CD 147 was also excellent as follow-up examinations after operation or drug administration.

### INDUSTRIAL APPLICABILITY

According to the method of the present invention, whether or not a sample provider has a high possibility of developing a colorectal cancer can be judged. Therefore, the method is useful because the sample provider can take a means of inhibiting the progression of cancer.

## Claims

1. A method for detecting a colorectal cancer, comprising the steps of:
measuring an amount of exosomes expressing CD 147 in a body fluid sample derived from a test individual with an anti-CD 147 monoclonal antibody or a fragment thereof; and
comparing a signal intensity of exosomes in said step with a signal intensity in a control individual,
wherein a case where said signal intensity in the test individual is found to be stronger than the signal intensity in the control individual serves as an
index of the presence of the colorectal cancer.

2. A method for detecting a colorectal cancer, comprising the steps of:
measuring an amount of exosomes expressing CD9 and/or CD63, and CD 147 in a body fluid sample derived from a test individual with
at least one member selected from the group consisting of an anti-CD9 monoclonal antibody and a fragment thereof and an anti-CD63 monoclonal antibody and a fragment thereof, and
an anti-CD 147 monoclonal antibody or a fragment thereof; and
comparing a signal intensity of exosomes in said step with a signal intensity in a control individual,
wherein a case where said signal intensity in the test individual is found to be stronger than the signal intensity in the control individual serves as an index of the presence of the colorectal cancer.

3. A method for evaluating treatment of a colorectal cancer, comprising the steps of:
measuring an amount of exosomes expressing CD9 and/or CD63, and CD 147 in a body fluid sample derived from a test individual after the treatment with
at least one member selected from the group consisting of an anti-CD9 monoclonal antibody and a fragment thereof and an anti-CD63 monoclonal antibody and a fragment thereof, and
an anti-CD 147 monoclonal antibody or a fragment thereof; and
comparing a signal intensity of exosomes in said step with a signal intensity in the test individual before the treatment,
wherein the method comprises the step of evaluating said treatment to have therapeutic effects to a colorectal cancer, in a case where the signal intensity after the treatment is found to be weaker than the signal intensity before the treatment.

4. The method according to any one of claims 1 to 3, wherein the step of measuring an amount of exosomes in the body fluid sample uses an ExoScreen method or a sandwich ELISA method.

5. The method according to any one of claims 2 to 4, wherein the anti-CD9 monoclonal antibody or a fragment thereof is a monoclonal antibody produced by hybridoma deposited under the accession number of FERM BP-11519.

6. The method according to any one of claims 2 to 4, wherein the anti-CD63 monoclonal antibody or a fragment thereof is a monoclonal antibody produced by hybridoma deposited under the accession number of FERM BP-11520, or a monoclonal antibody produced by hybridoma deposited under the accession number of FERM BP-11521.

7. A kit for use in a method as defined in any one of claims 1 to 6, wherein the kit comprises an anti-CD 147 monoclonal antibody or a fragment thereof.

8. A kit for use in a method as defined in any one of claims 2 to 6, wherein the kit comprises an anti-CD9 monoclonal antibody and/or an anti-CD63 monoclonal antibody or fragments thereof, and an anti-CD 147 monoclonal antibody or a fragment thereof.

9. Use of exosomes as a marker of a colorectal cancer, recognized by
at least one member selected from the group consisting of an anti-CD9 monoclonal antibody and a fragment thereof and an anti-CD63 monoclonal antibody and a fragment thereof, and
an anti-CD 147 monoclonal antibody or a fragment thereof.

10. A method for providing information for a colorectal cancer or a suspect of a colorectal cancer, **characterized in that** the method comprises detecting exosomes recognized by
at least one member selected from the group consisting of an anti-CD9 monoclonal antibody and a fragment thereof and an anti-CD63 monoclonal antibody and a fragment thereof, and
an anti-CD 147 monoclonal antibody or a fragment thereof from a body fluid sample derived from a test individual.
